# EUROPEAN PATENT SPECIFICATION

(11) **EP 4 125 871 B1**
(45) Date of publication and mention of the grant of the patent: **02.09.2026**
(21) Application number: 21716045.6
(22) Date of filing: 12.03.2021
(51) Int. Cl.: A61K 31/357, A61P 11/00

(54) **TREATING PULMONARY INFLAMMATORY DISEASE ASSOCIATED WITH COVID-19 BY ADMINISTERING RESINIFERATOXIN**
BEHANDLUNG VON ENTZÜNDLICHEN LUNGENERKRANKUNGEN IM ZUSAMMENHANG MIT COVID-19 DURCH VERABREICHUNG VON RESINIFERATOXIN
TRAITER LES MALADIES INFLAMMATOIRES PULMONAIRES ASSOCIÉES AU COVID-19 PAR L'ADMINISTRATION DE RÉSINIFÉRATOXINE

(30) Priority: 30.03.2020 US 202063002165 P; 08.12.2020 US 202063122858 P
(43) Date of publication of application: 08.02.2023
(73) Proprietor: Vivasor, Inc., San Diego, CA 92121 (US); Board of Regents of the University of Nebraska, Lincoln, NE 68583 (US)
(72) Inventor: NAHAMA, Alexis, San Diego, CA 92121 (US); JI, Henry Hongjun, San Diego, CA 92121 (US); ZUCKER, Irving H., Lincoln, NE 68583 (US); WANG, Hanjun, Lincoln, NE 68583 (US)
(74) Representative: Script Intellectual Property LLP
(86) International application number: PCT/US2021/022089
(87) International publication number: WO 2021/202084

(56) References cited:
- ELEKES ET AL: "Role of capsaicin-sensitive afferents and sensory neuropeptides in endotoxin-induced airway inflammation and consequent bronchial hyperreactivity in the mouse", REGULATORY PEPTIDES, ELSEVIER SCIENCE BV, NL, vol. 141, no. 1-3, 29 March 2007 (2007-03-29), pages 44 - 54, XP022003436, ISSN: 0167-0115, DOI: 10.1016/J.REGPEP.2006.12.018

## Description

### CROSS-REFERENCE TO RELATED APPLICATIONS

This application claims priority to U.S. Provisional Application No. 63/002,165, filed on March 30, 2020, and U.S. Provisional Application No. 63/122,858, filed on December 8, 2020, the disclosures of each of which are hereby incorporated by reference in their entireties.

Throughout this application various publications, patents, and/or patent applications are referenced.

### TECHNICAL FIELD

The present disclosure provides a method for treating pulmonary inflammatory disease comprising administering an effective amount of resiniferatoxin (RTX) by an epidural, peri-ganglionic or an intra-ganglionic administration.

### BACKGROUND

RTX acts as an ultrapotent analog of capsaicin, the pungent principal ingredient of the red pepper. RTX is a tricyclic diterpene isolated from certain species of *Euphorbia.* A homovanillyl group is an important structural feature of capsaicin and is the most prominent feature distinguishing resiniferatoxin from typical phorbol-related compounds. Native RTX has the following structure:

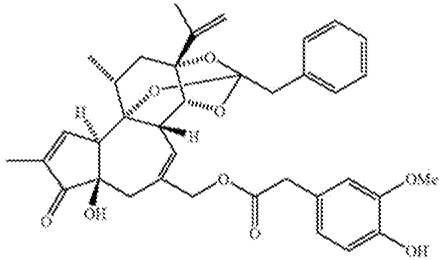

RTX and analog compounds such as tinyatoxin and other compounds (20-homovanillyl esters of diterpenes such as 12-deoxyphorbol 13-phenylacetate 20-homovanillate and mezerein 20-homovanillate) are described in U.S. Patent Nos. 4,939,194; 5,021,450; and 5,232,684. Other resiniferatoxin-type phorboid vanilloids have also been identified (Szallasi et al. (1999) Brit. J. Pharmacol. 128:428-434)*.*

RTX is known as a TRPV1 agonist. TRPV1, the transient receptor potential cation channel subfamily V member 1 (also known as Vanilloid receptor-1 (VR1)) is a multimeric cation channel prominently expressed in nociceptive primary afferent neurons (Caterina et al. (1997) Nature 389:816-824; Tominaga et al. (1998) Neuron 21:531-543). Activation of TRPV1 typically occurs at the nerve endings via application of painful heat and is up regulated during certain types of inflammatory stimuli. Activation of TRPV1 in peripheral tissues by a chemical agonist results in the opening of calcium channels and the transduction of a pain sensation (Szalllasi et al. (1999) Mol. Pharmacol. 56:581-587). However, direct application of certain TRPV1 agonists to the cell body of a neuron (ganglion) expressing TRPV1 opens calcium channels and triggers a cascade of events leading to programmed cell death ("apoptosis") (Karai et al. (2004) J. of Clin. Invest. 113:1344-1352)*.*

Respiratory failure due to acute respiratory distress syndrome (ARDS) is one of the major causes of mortality (53%) associated with infection with the novel coronavirus SARS-CoV-2 (COVID-19 disease) (Ruan et al. (2020) Intensive Care Med Mar 3:10-3) and can also result from other diseases and disorders, including other viral diseases or lung injury. Around 10% of the patients require intensive care unit (ICU) care with ventilatory support and an ICU mortality rate of 79% has been reported (Huang et al. (2020) Lancet Vol. 394, Issue 10233, P497-506).

Coronaviruses are a group of viruses that causes diseases in birds, mammals and humans. The diseases include respiratory infections and enteric infections which can be mild or lethal. Coronaviruses are viruses in the subfamily *Orthocoronavirinae,* in the family *Coronaviridae,* in the order Nidovirales. The genus Coronavirus includes avian infectious bronchitis virus, bovine coronavirus, canine coronavirus, human coronavirus 299E, human coronavirus OC43, murine hepatitis virus, rat coronavirus, and porcine hemagglutinating encephalomyelitis virus. The genus Torovirus includes Berne virus and Breda virus. Coronaviruses are enveloped viruses having a positive-sense single-stranded RNA genome and a nucleocapsid of helical symmetry. The genomic size of coronaviruses ranges from approximately 26 to 32 kilobases, which is believed to be the largest for an RNA virus. It is interesting to note that the 2019-2020 China pneumonia outbreak in Wuhan was traced to a novel coronavirus, labeled 2019-nCoV by the World Health Organization (WHO), and also known as SARS-CoV-2, which causes Coronavirus disease 2019, or COVID-19.

ARDS was first described in 1967 (Ashbaugh et al. (1967) Lancet 2:319-323) and is characterized by diffuse pulmonary microvascular injury resulting in increased permeability and hypoxemiea caused by intrapulmonary shunts. The first two stages of ARDS progression (i.e., 12-72 hours after onset) is a critical window for intervention as the syndrome can be reversed if the initiating factors and the inflammatory mediators can be controlled. An early diagnosis may also be facilitated if the initiating stimulus is known as in determination of sepsis, aspiration of gastric contents, multiple transfusions, severe fractures, burns, pancreatitis or severe trauma. Upon progression to a third stage of ARDS pulmonary hypertension increases, heart rate increases to compensate for hypoxemia and mechanical ventilation supportive therapy is generally required. Pathologically the cellular infiltrates are denser with continued neutrophil infiltration and increasing mononuclear, lymphocyte and fibroblast cell infiltrates.

The severity of the disease is higher in older patients with 80% death observed in those over 60-65 years of age (CDC COVID-19 Response Team (2020) MMWR Morb Mortal Wkly Rep 69:343-346), while younger infected seem to be less susceptible and exhibit medium-mild symptoms (Wu et al. (2020) JAMA Published online February 24, 2020). Once the lower respiratory tract is affected, the respiratory distress progresses very quickly, with time to death reported as rapidly as 14 days from initial symptoms despite availability of ventilator palliative support. It has been proposed that the severity and mortality rates of the susceptible population infected by COVID-19 is related to a cytokine storm, in which an exaggerated production of pro-inflammatory substances are released into the pulmonary microenvironment over a short period of time (Mehta et al. (2020) Lancet Vol. 395, Issue 10229, P1033-1034). ELEKES ET AL: "Role of capsaicin-sensitive afferents and sensory neuropeptides in endotoxin-induced airway inflammation and consequent bronchial hyperreactivity in the mouse", REGULATORY PEPTIDES, ELSEVIER SCIENCE BV, NL, vol. 141, no. 1-3, 29 March 2007 (2007-03-29), pages 44-54 discloses the use of resiniferatoxin (RTX) in an endotoxin-induced airway inflammation model in mice.

Novel life-saving strategies are desperately needed to mitigate the high mortality that is associated with acute respiratory distress, including such distress associated with late stage viral infection.

### SUMMARY

The present invention concerns a composition comprising resiniferatoxin (RTX) for use in treating pulmonary inflammatory disease in a subject according to claim 1. The present disclosure provides a method for treating pulmonary inflammatory diseases comprising administering an effective amount of resiniferatoxin (RTX) by an epidural, peri-ganglionic or an intra-ganglionic administration. In some embodiments, the dose of RTX for an adult human is from about 0.1 µg to about 100 µg.

Embodiment 1 is a method for treating pulmonary inflammatory disease comprising administering to a subject in need of treatment for pulmonary inflammatory disease an effective amount of resiniferatoxin (RTX) epidurally, peri-ganglionically or intra-ganglionically.

Embodiment 2 is a composition comprising resiniferatoxin (RTX) for use in a method of treating a subject in need of treatment for pulmonary inflammatory disease.

Embodiment 3 is the composition for use of embodiment 2, wherein the method comprises administering the composition to the subject epidurally, peri-ganglionically or intra-ganglionically.

Embodiment 4 is the method of embodiment 1 or the composition for use of embodiment 2 or 3, wherein the effective amount of RTX results in a reduction in one or more cytokines comprising IL-6, IL-1 β and/or IFNγ.

Embodiment 5 is the method or composition for use of any one of the preceding embodiments, wherein the effective amount of RTX results in improved pulmonary function.

Embodiment 6 is the method or composition for use of any one of the preceding embodiments, wherein the effective amount of RTX results in reduced lung edema.

Embodiment 7 is the method or composition for use of any one of the preceding embodiments, wherein the subject is an adult human.

Embodiment 8 is the method or composition for use of any one of the preceding embodiments, wherein the RTX is administered in a dose of from about 0.1 µg to about 100 µg.

Embodiment 9 is the method or composition for use of embodiment 8, wherein the dose is from about 0.1 µg to about 1 µg, about 1 µg to about 5 µg, about 5 µg to about 10 µg, about 10 µg, to about 20 µg, about 20 µg to about 50 µg, or about 50 to about 100 µg.

Embodiment 10 is the method or composition for use of any one of the preceding embodiments, wherein the method comprises epidural administration.

Embodiment 11 is the method or composition for use of any one of embodiments 1-9, wherein the method comprises a peri-ganglionic nerve block.

Embodiment 12 is the method or composition for use of any one of embodiments 1-9, wherein the method comprises intra-ganglionic administration.

Embodiment 13 is the method or composition for use of any one of the preceding embodiments, wherein the RTX is administered in a pharmaceutical formulation comprising the RTX and a pharmaceutically acceptable carrier.

Embodiment 14 is the method or composition for use of embodiment 13 wherein the pharmaceutically acceptable carrier comprises water.

Embodiment 15 is the method or composition for use of embodiment 13, wherein the pharmaceutically acceptable carrier comprises saline.

Embodiment 16 is the method or composition for use of any one of embodiments 13-15, wherein the RTX is present in the pharmaceutical formulation at a concentration ranging from 1 µg/ml to 100 µg/ml.

Embodiment 17 is the method or composition for use of embodiment 16, wherein the RTX is present in the pharmaceutical formulation at a concentration ranging from 1 µg/ml to 5 µg/ml, 5 µg/ml to 10 µg/ml, 10 µg/ml to 20 µg/ml, 20 µg/ml to 50 µg/ml, or 50 µg/ml to 100 µg/ml.

Embodiment 18 is the method or composition of any one of the preceding embodiments, wherein the pulmonary inflammatory disease is selected from the group consisting of acute respiratory distress syndrome (ARDS), chronic obstructive pulmonary disease (COPD), pulmonary arterial hypertension (PAH), chronic inflammatory lung disease, pulmonary fibrosis, pulmonary vasculitis, pulmonary sarcoidosis, inflammation and/or infection associated with lung transplantation, acute or lung rejection and/or dysfunction, bronchitis, sinusitis, asthma, cystic fibrosis, bacterial infection, fungal infection, parasite infection, viral infection, bronchiolitis obliterans syndrome (BOS), primary ciliary dyskinesia (PCD), alveolar proteinosis, idiopathic pulmonary fibrosis (IPF), eosinophilic pneumonia, eosinophilic bronchitis, inflammation and/or infection associated with mechanical ventilation, ventilator-associated pneumonia, asbestos-related airway disorder or disease, dust-related airway disorder or disease, silicosis, and radiation or chemical agent-related airway disease or disorder, and any combination thereof.

Embodiment 19 is the method or composition of any one of the preceding embodiments, wherein the pulmonary inflammatory disease is acute respiratory distress syndrome (ARDS).

Embodiment 20 is the method or composition of any one of the preceding embodiments, wherein the pulmonary inflammatory disease is chronic obstructive pulmonary disease (COPD).

Embodiment 21 is the method or composition of any one of the preceding embodiments, wherein the pulmonary inflammatory disease is pulmonary arterial hypertension (PAH).

Embodiment 22 is the method or composition of any one of the preceding embodiments, wherein the pulmonary inflammatory disease is inflammation and/or infection associated with mechanical ventilation and/or ventilator-associated pneumonia.

Embodiment 23 is the method or composition of any one of the preceding embodiments, wherein the pulmonary inflammatory disease is associated with COVID-19.

### BRIEF DESCRIPTION OF THE DRAWINGS

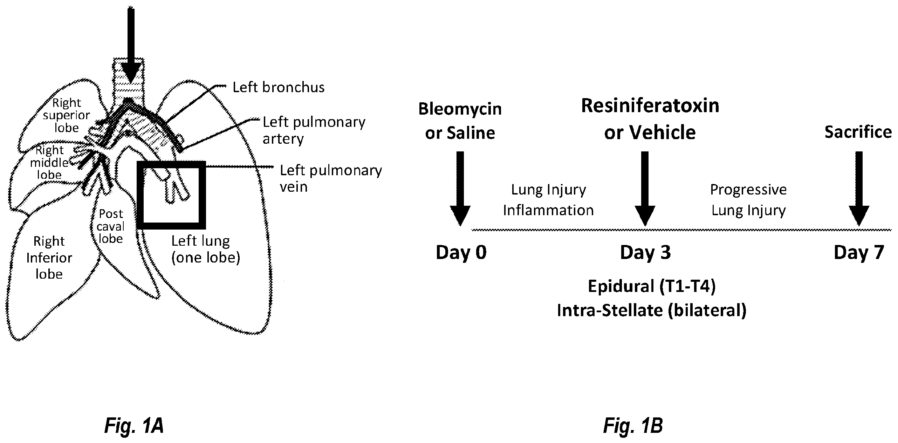

FIG. 1A-B show a schematic diagram of the study design (FIG. 1A) and the treatment plan with a timeline (FIG. 1B). In FIG. 1A, the arrow indicates that bleomycin (Bleo) (2.5 mg/kg, ~0.15 mL) was administered intra-tracheally to the lungs. The square shows the location where lung tissue was collected for cytokine measurement. As shown in FIG. 1B - at day 0, Bleo or saline was given intra-tracheally; at day 3, resiniferatoxin (RTX) or vehicle (Veh) was given into epidural space or into stellate ganglia; at day 7, the rats were sacrificed.

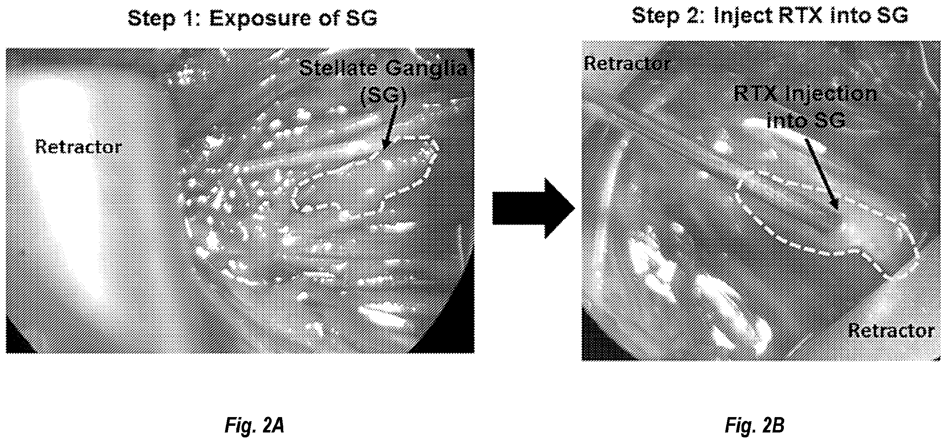

FIG. 2A-B show the procedure for stellate isolation and administration of Veh or RTX. FIG. 2A shows step 1 of the procedure - stellate ganglia was exposed. The arrow shows that stellate ganglion was located medially to the origins of internal thoracic and costocervical arteries. FIG. 2B shows step 2 of the procedure - RTX (5 µL, 50 mg/mL) was injected into the left and right stellate ganglions. The arrow shows the tip of a 5-µL syringe inside the stellate ganglion.

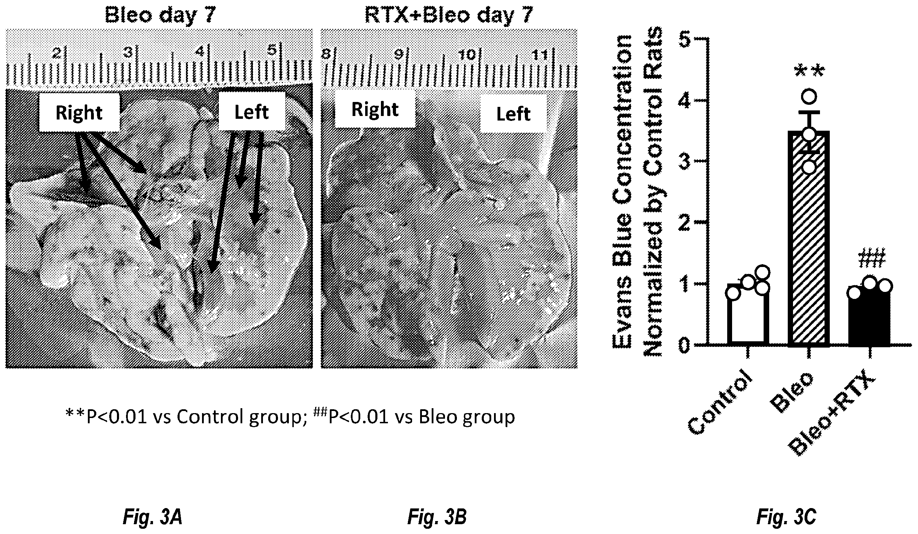

FIG. 3A-C show plasma extravasation was reduced following epidural RTX treatment at the 7-day time point after Bleo administration. FIG. 3A-B shows representative images of the lungs from the Bleo group (FIG. 3A) and the Bleo+RTX group (FIG. 3B). FIG. 3C shows Evans blue concentration from Control, Bleo, and Bleo+RTX groups. ***P*<0.01 vs. Control. ^{*#*#}*P*<0.01 vs. Bleo.

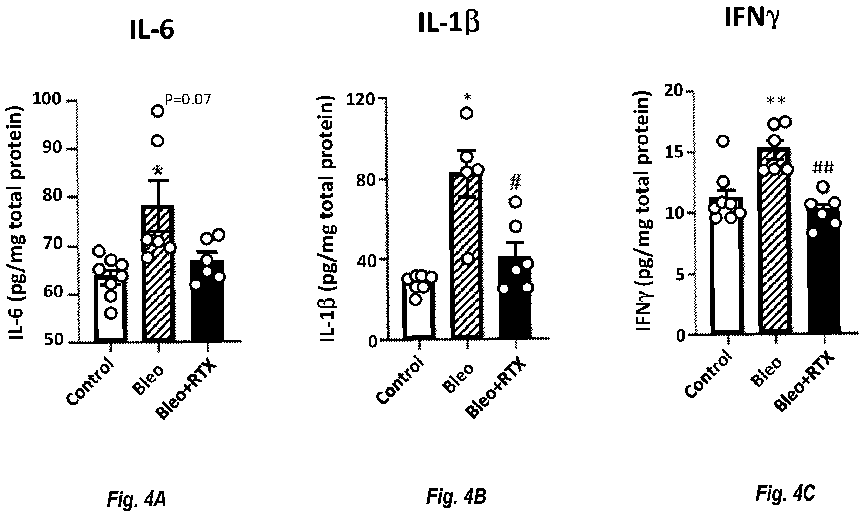

FIG. 4A-C show day 7 lung tissue cytokine levels following day 3 Veh or epidural RTX administration. FIG. 4A shows interleukin 6 (IL-6). FIG. 4B shows interleukin 1β (IL-1β). FIG. 4C shows interferon γ (IFNγ). **P*<0.05 and ***P*<0.01 vs. Control. ^{#}*P*<0.05 and *^{##}P*<0.01 vs. Bleo.

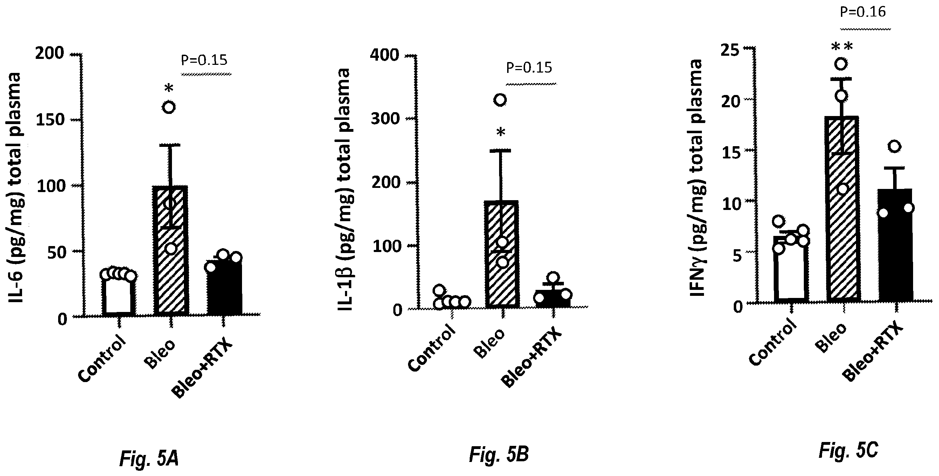

FIG. 5A-C show day 7 plasma cytokine levels following day 3 Veh or epidural RTX administration.

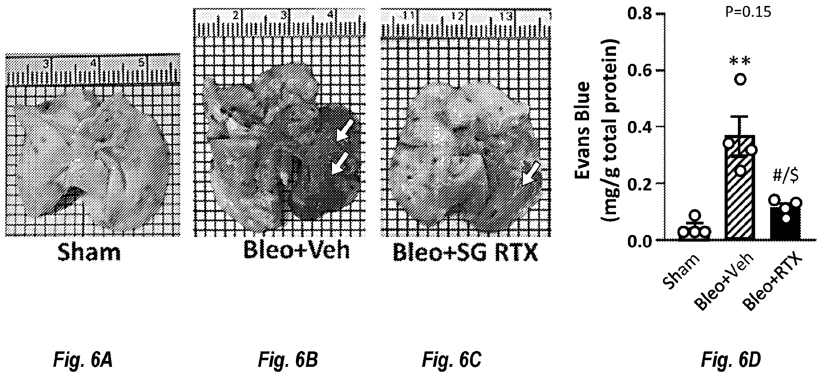

FIG. 6A-D show Evans blue extravasation was reduced following stellate RTX injection at the 7-day time point after Bleo administration. FIG. 6A-C show representative images of the lungs from Sham (FIG. 6A), Bleo+Veh group (FIG. 6B), and Bleo+RTX group (FIG. 6C). Arrows point to areas of Evans blue extravasation. FIG. 6D shows mean Evans blue concentration from each group. ***P*<0.01 vs. Sham. ^{#}*P*<0.05 vs. Bleo+Veh. ^{$}*P*<0.05 vs. Sham.

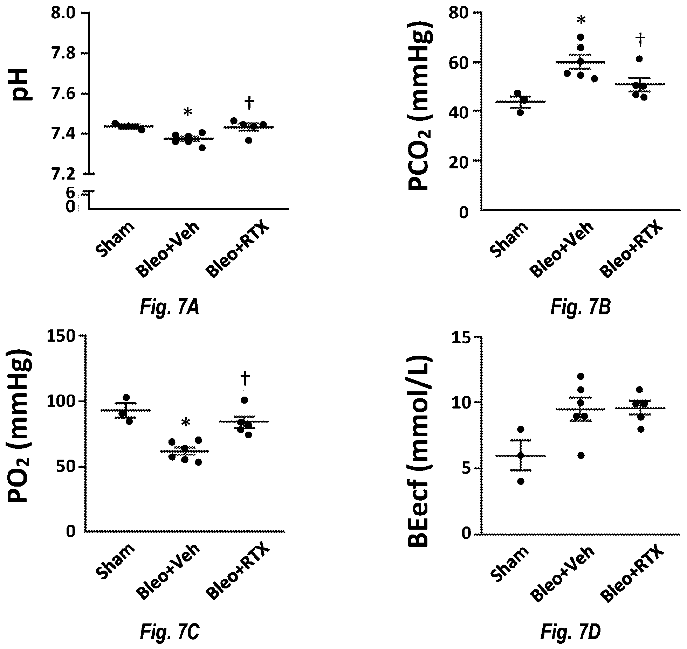

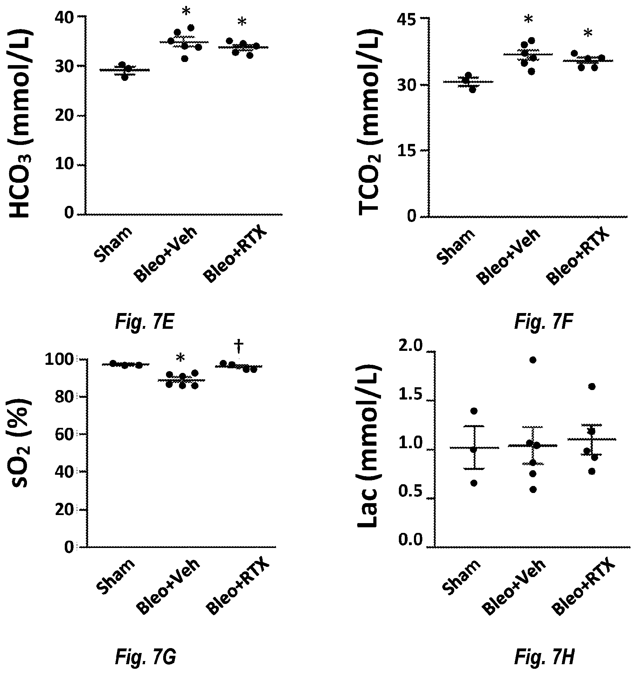

FIG. 7A-H show day 7 arterial blood gases in Sham, Bleo + Veh and Bleo + RTX rats following intra-stellate administration at day 3 post-injury. FIG. 7A shows pH. FIG. 6B shows partial pressure of carbon dioxide (PCO₂). FIG. 7C shows partial pressure of oxygen (PO₂). FIG. 7D shows base excess (BE). FIG. 7E shows bicarbonate (HCO₃). FIG. 7F shows total CO₂ (TCO₂). FIG. 7G shows oxygen saturation (sO₂). FIG. 7H shows lactate (Lac). **P*<0.05 vs. Sham. †, *P*<0.05 vs. Bleo+Veh.

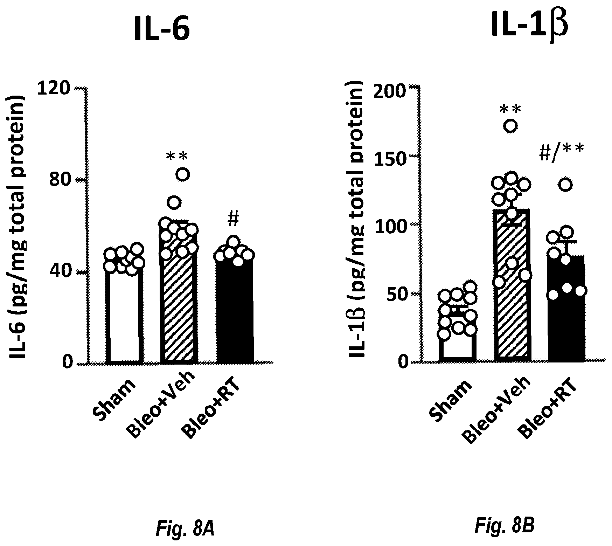

FIG. 8A-B show day 7 lung tissue cytokine levels following day 3 Veh or stellate RTX administration. FIG. 8A shows IL-6. FIG. 8B shows IL-1β.

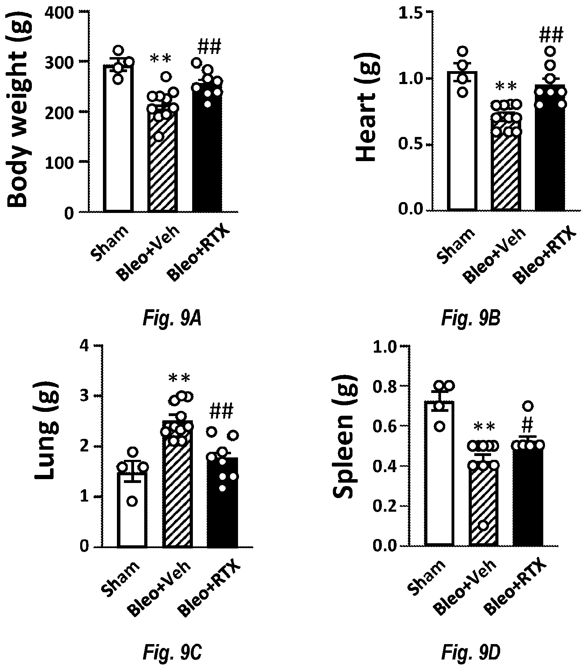

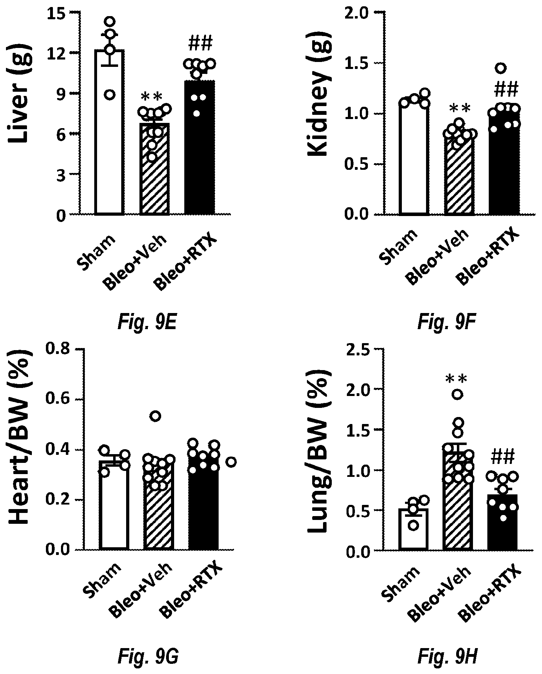

FIG. 9A-H show body weight (BW) and individual organ weight among groups. FIG. 9A shows body weight. FIG. 9B shows heart. FIG. 9C shows lung. FIG. 9D shows spleen. FIG. 9E shows liver. FIG. 9F shows kidney. FIG. 9G shows heart/BW. FIG. 9H shows lung/BW. Compared to sham rats, wet lung weight (WLW) as well as the ratio of WLW to BW was significantly higher in the Bleo+Veh rats, which was significantly reduced by intra-stellate injection of RTX. These data suggest that intra-stellate injection of RTX reduces lung edema post Bleo.

### DETAILED DESCRIPTION

Reference will now be made in detail to certain embodiments of the invention, examples of which are illustrated in the accompanying drawings.

Before describing the present teachings in detail, it is to be understood that the disclosure is not limited to specific compositions or process steps, as such may vary. It should be noted that, as used in this specification and the appended claims, the singular form "a", "an" and "the" include plural references unless the context clearly dictates otherwise. Thus, for example, reference to "a conjugate" includes a plurality of conjugates and reference to "a cell" includes a plurality of cells and the like. It is understood the use of the alternative (e.g., "or") herein is taken to mean either one or both or any combination thereof of the alternatives.

The term "and/or" used herein is to be taken mean specific disclosure of each of the specified features or components with or without the other. For example, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

As used herein, terms "comprising", "including", "having" and "containing", and their grammatical variants, as used herein are intended to be non-limiting so that one item or multiple items in a list do not exclude other items that can be substituted or added to the listed items. It is understood that wherever aspects are described herein with the language "comprising," otherwise analogous aspects described in terms of "consisting of" and/or "consisting essentially of" are also provided.

As used herein, the term "about" refers to a value or composition that is within an acceptable error range for the particular value or composition as determined by one of ordinary skill in the art, which will depend in part on how the value or composition is measured or determined, i.e., the limitations of the measurement system. For example, "about" or "approximately" can mean within one or more than one standard deviation per the practice in the art. Alternatively, "about" or "approximately" can mean a range of up to 10% (i.e., ±10%) or more depending on the limitations of the measurement system. For example, about 5 mg can include any number between 4.5 mg and 5.5 mg. Furthermore, particularly with respect to biological systems or processes, the terms can mean up to an order of magnitude or up to 5-fold of a value. When particular values or compositions are provided in the instant disclosure, unless otherwise stated, the meaning of "about" or "approximately" should be assumed to be within an acceptable error range for that particular value or composition. In some embodiments, "about" encompasses variation within 10%, 5%, 2%, 1%, or 0.5% of a stated value.

Numeric ranges are inclusive of the numbers defining the range. Measured and measurable values are understood to be approximate, taking into account significant digits and the error associated with the measurement. Also, all ranges are to be interpreted as encompassing the endpoints in the absence of express exclusions such as "not including the endpoints"; thus, for example, "ranging from 1 to 10" includes the values 1 and 10 and all integer and (where appropriate) non-integer values greater than 1 and less than 10.

The use of "comprise", "comprises", "comprising", "contain", "contains", "containing", "include", "includes", and "including" are not intended to be limiting. It is to be understood that both the foregoing general description and detailed description are exemplary and explanatory only and are not restrictive of the teachings. Unless specifically noted in the above specification, embodiments in the specification that recite "comprising" various components are also contemplated as "consisting of" or "consisting essentially of" the recited components; embodiments in the specification that recite "consisting of" various components are also contemplated as "comprising" or "consisting essentially of" the recited components; and embodiments in the specification that recite "consisting essentially of" various components are also contemplated as "consisting of" or "comprising" the recited components (this interchangeability does not apply to the use of these terms in the claims).

### Definitions

As used herein, "pulmonary inflammatory disease" is used collectively to refer to those acute and chronic pathological conditions associated with inflammatory processes. Non-limiting examples of pulmonary inflammatory disease includes acute respiratory distress syndrome (ARDS), pneumonia, pneumonitis, bronchitis, lung infections, atelactasis, conditions associated with inflammatory lung injuries such as chemotherapeutic (e.g., bleomycin) induced lung injury, pancreatitis induced lung injury, hyperoxia induced lung injury, amiodarone induced pneumonitis, radiation pneumonitis, chlorine gas or smoke inhalation injuries, bronchiolitis obliterans/obstructive pneumonia (BOOP), viral and mycoplasmal pneumonias (e.g., Legionella and CMV lung), pneumoconioses, pulmonary vasculitis, pulmonary sarcoidosis, airways bacterial infection, airways fungal infection, airways parasite infection, airways viral infection, mechanical ventilation-associated inflammation and/or infection, ventilator-associated pneumonias. Non-limiting examples of chronic pathological conditions of the lung include chronic obstructive pulmonary disease (COPD), pulmonary arterial hypertension (PAH), cystic fibrosis, silicosis, asbestosis, asthma, atherosclerosis, chronic bronchitis, chronic inflammation due to chronic bacterial or viral infections, coronary artery disease, idiopathic pulmonary fibrosis (IPF), familial pulmonary fibrosis (FPF), desquamative interstitial pneumonitis (DIP), hypersensitivity pneumonitis, interstitial pneumonitis, collagen vascular disease, sarcoidosis, coal worker's pneumoconiosis, bronchopulmonary dysplasia, inflammatory pseudotumor.

As used herein, "epidural administration" refers to delivery of a drug or pharmaceutical formulation into the epidural space (also known as "extradural space" or "peridural space") which is the outermost part of the spinal canal. It is the space within the canal (formed by the surrounding vertebrae) lying outside the dura mater (which encloses the arachnoid mater, subarachnoid space, the cerebrospinal fluid, and the spinal cord). For example, epidural delivery may include delivery to the epidural space without direct injection into nerves or may include epidural delivery into nerve tissue.

As used herein, "peri-ganglionic administration" refers to delivery of a drug or pharmaceutical formulation into the space surrounding a ganglion.

"Intra-ganglionic administration" means administration to a ganglion. Intra-ganglionic administration can be achieved by direct injection into the ganglion and also includes selective nerve root injections, in which the compound passes up the connective tissue sleeve around the nerve and enters the ganglion from the nerve root just outside the vertebral column.

The terms "effective amount", "therapeutically effective amount" or "effective dose" or related terms may be used interchangeably and refer to an amount of the therapeutic agent that when administered to a subject, is sufficient to affect a measurable improvement or prevention of a pulmonary inflammatory disease. For example, administering an effective dose may improve pulmonary function expressed as partial pressure of CO₂ (pCO₂), partial pressure of O₂ (pO₂), and oxygen saturation (sO₂) when measured in arterial blood. In another example, an effective dose may reduce lung edema. Therapeutically effective amounts of the therapeutic agents provided herein, when used alone or in combination with an antiviral agent, will vary depending upon the relative activity of the therapeutic agent, and depending upon the subject and disease condition being treated, the weight and age and sex of the subject, the severity of the disease condition in the subject, the manner of administration and the like, which can readily be determined by one of ordinary skill in the art. In one embodiment, a therapeutically effective amount will depend on certain aspects of the subject to be treated and the disorder to be treated and may be ascertained by one skilled in the art using known techniques. In addition, as is known in the art, adjustments for age as well as the body weight, general health, sex, diet, time of administration, drug interaction, and the severity of the disease may be necessary.

The terms "subject" and "patient" as used herein refer to human and non-human animals, including vertebrates, mammals and non-mammals. In one embodiment, the subject can be human, non-human primates, simian, ape, murine (e.g., mice and rats), bovine, porcine, equine, canine, feline, caprine, lupine, ranine or piscine.

The term "administering", "administered" and grammatical variants refers to the physical introduction of a therapeutic agent to a subject, using any of the various methods and delivery systems known to those skilled in the art. Exemplary routes of administration for the formulations disclosed herein include intravenous, intramuscular, subcutaneous, intraperitoneal, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, as well as in vivo electroporation. In one embodiment, the formulation is administered via a non-parenteral route, e.g., orally. Other non-parenteral routes include a topical, epidermal or mucosal route of administration, for example, intranasally, vaginally, rectally, sublingually or topically. Administering can also be performed, for example, once, a plurality of times, and/or over one or more extended periods.

"Treating" is to be understood broadly and encompasses any beneficial effect, including, e.g., delaying, slowing, or arresting the worsening of symptoms associated with pulmonary inflammatory disease or remedying such symptoms, at least in part. Treating also encompasses bringing about any form of improved patient function, as discussed in detail below. In some embodiments, treatment also means prolonging survival as compared to expected survival if not receiving treatment. Those in need of treatment include those who already have the disease or disorder, as well as those who tend to have the disease or disorder or who should prevent the disease or disorder.

A "pharmaceutically acceptable vehicle" for therapeutic purposes is a physical embodiment that can be administered to a subject. Pharmaceutically acceptable vehicles include pills, capsules, caplets, tablets, oral fluids, injection fluids, sprays, aerosols, troches, dietary supplements, creams, lotions, oils, solutions, pastes, powders, steam, Or it may be a liquid, but is not limited to these. An example of a pharmaceutically acceptable vehicle is a buffered isotonic solution such as phosphate buffered saline (PBS).

It has been observed that the clinical signs of COVID-19 are consistent with those observed in viral pneumonia. These pulmonary changes are likely responsible for both systemic and localized immune response leading to a hyperinflammatory state. The mortality rate in patients is suspected to be related to virally driven cytokine storm similar to that seen in SARS-CoV-2 infections. The cytokine storm is a result of a severe immune reaction in the lungs as measured by high levels of inflammatory markers (c-reactive protein, serum ferritin) and cytokine levels (IL-6, IL-2, IL-7, IL-10, GSCF, IP10, MCP1, MIP1A, IL-1β, IFNγ, and TNFα) in the plasma. ICU patients have higher plasma levels of IL-2, IL-7, IL-10, GSCF, IP10, MCP1, MIPIA, and TNFα as compared to non-ICU patients, indicating that the presence of high circulating cytokine levels is associated with the severity of the disease. It is therefore necessary to interfere with the inflammatory cascade at a higher level (i.e., eliminating the pro-inflammatory efferent pathway) to appropriately control the multimodal aspect of this inflammatory process.

The morbidity, severity of the disease, and underlying physiological events linked to mortality can be explained by the involvement of the TRPV1 expressing neuronal system (afferent/efferent neurons). TRPV1 positive pathways are responsible for pain transmission, inflammation and immunomodulation throughout the entire pulmonary system.

The afferent innervation of the pulmonary system is mainly conducted by the vagal nerve and its branches. TRPV1 expressing C-fibers are small diameter unmyelinated fibers in the vagal nerve and responsible for several processes in the airways and lungs. Afferent fibers innervating pulmonary structure are also carried by sympathetic fibers with cell bodies located in the dorsal root ganglia of the thoracic segment between T1 and T6. The activation of this thoracic segment has been related to severe pneumonitis.

RTX is an ultra-potent agonist of the TRPV1 receptor, and it works by inducing neurolysis of TRPV1-expressing neurons in dorsal root ganglia (DRG), dorsal horns (DH) of the spinal cord, or peripheral nerve ending when applied locally as a nerve block. The strong binding of RTX to TRPV1 receptors forces the opening of the channel gates leading to a slow and sustained increase in intracellular Ca2+, which in turn disrupts the intra-cellular mitochondrial metabolism and results in neural cell or nerve fiber deletion within minutes. The inventors have discovered a therapeutic use of RTX, an ultra-potent TRPV1 agonist, as an ablating agent of TRPV1 positive pulmonary pathways in patients with acute pulmonary inflammatory disease. Such a therapeutic approach targeting TRPV1 expressing neurons in the lungs modulates the inflammatory and immune signal activity, leading to reduced mortality and better overall outcomes.

### Exemplary Compositions for Use and Related Methods

Provided herein are methods of treating and compositions for use in treating pulmonary inflammatory disease in which RTX is delivered epidurally, peri-ganglionically via nerve block or intra-ganglionically. In various embodiments, the route of administration for an ablative agent such as RTX includes thoracic epidural injections, peri-ganglionic nerve block or intra-ganglionic injections for "chemical" targeted lung denervation. In one embodiment, RTX is administered by accessing the vagal nerve with a local ablative agent through the neck, going low and away from the carotid bulb. The nerve location could then be confirmed using ultrasound guidance. In some embodiments, RTX is delivered peri-ganglionically to the stellate ganglion. In some embodiments, RTX is delivered intra-ganglionically to the stellate ganglion.

In some embodiments, an epidural or peri-ganglionic injection of RTX in subjects with advanced COVID-19 disease supports palliative ventilation therapy by ablating afferent nerves at the thoracic DRG level to increase survival.

In some embodiments, the effective amount of RTX results in a reduction in one or more cytokines comprising IL-6, IL-1 β and/or IFNγ. In some embodiments, the effective amount of RTX results in improved pulmonary function, such as higher pO₂ or sO₂, or lower pCO₂. In some embodiments, the effective amount of RTX results in reduced lung edema. Such reductions or improvements may occur relative to the condition of the subject prior to the administration of RTX.

The methods described herein are for use with any subject in whom RTX is effective, e.g., able to bind and activate TRPV1 or a homolog thereof, and who is in need of treatment for PD. In some embodiments, the RTX is administered at a dose of 0.1-100 µg. In some embodiments, the dose of RTX ranges from 0.1-0.5 µg, 0.5-1 µg, 1-2 µg, 2-5 µg, 5-10 µg, 10-20 µg, 20-30 µg, 30-40 µg, 40-50 µg, 50-60 µg, 60-70 µg, 70-80 µg, 80-90 µg, or 90-100 µg. In some embodiments, a 2-, 3-, or 4-point peri-ganglionic nerve block technique is used, with a total dosage in any of the ranges listed above, such as a total dosage of 0.5-1 µg, 1-2 µg, 2-5 µg, 5-10 µg, 10-15 µg, 15-20 µg, or 20-25 µg.

The dosage can be adjusted depending on the proximity of the site of administration to the nerve fiber. For example, where ultrasound or a nerve stimulator is used to ensure that the site of administration is very close to the nerve, a lower dose and/or volume can be used. Alternatively, a nerve block can be accomplished using a larger volume to ensure contact with the desired nerves. Notably, RTX is specific for the TRPV1 receptor and therefore does not affect non-target nerves such as motor neurons that do not have enough TRPV1 receptors to be sensitive to RTX.

Multiple examples of formulations of RTX are available in the literature. See, e.g., Ueda et al. (2008) J. of Cardiovasc. Pharmacol. 51:513-520, and US 2015/0190509 A1. Any suitable formulation of RTX for parenteral administration (e.g., injection) may be used.

In some embodiments, the RTX, which may be at the dosages discussed above, is administered with a pharmaceutically acceptable carrier. In some embodiments, the pharmaceutically acceptable carrier comprises water. In some embodiments, the pharmaceutically acceptable carrier comprises polysorbate 80. In some embodiments, the pharmaceutically acceptable carrier comprises polyethylene glycol. In some embodiments, the pharmaceutically acceptable carrier comprises sugar or sugar alcohol. In some embodiments, the pharmaceutically acceptable carrier comprises mannitol. In some embodiments, the pharmaceutically acceptable carrier comprises dextrose. In some embodiments, the pharmaceutically acceptable carrier comprises a pharmaceutically acceptable buffer. In some embodiments, the pharmaceutically acceptable carrier comprises a phosphate buffer. In some embodiments, the pharmaceutically acceptable carrier comprises a pharmaceutically acceptable salt. In some embodiments, the pharmaceutically acceptable carrier comprises NaCl. In some embodiments, the pharmaceutically acceptable carrier comprises an organic solvent such as ethanol or DMSO, e.g., as a minority or residual component used as an aid in dissolving RTX before dilution in a primarily aqueous composition.

The concentration of RTX in the formulation may be any suitable value for delivery of the intended dose. In some embodiments, the concentration of RTX in the pharmaceutical formulation is in the range of 0.1 to 300 µg/ml. In some embodiments, the concentration of RTX in the pharmaceutical formulation is in the range of 0.1-1 µg/ml, 1-5 µg/ml, 5-10 µg/ml, 10-20 µg/ml, 10-30 µg/ml, 20-30 µg/ml, 20-50 µg/ml, 50-100 µg/ml, 100-150 µg/ml, 150-200 µg/ml, 200-250 µg/ml, or 250-300 µg/ml. In some embodiments, the concentration of RTX in the pharmaceutical formulation is in the range of 5-50 µg/ml, or 8-25 µg/ml.

Starting from a concentrated stock solution, a formulation of RTX for delivery into a subject may be prepared by dilution in an appropriate diluent, such as saline.

The formulation may have any pH suitable for intra-articular administration. In some embodiments, the pharmaceutical formulation comprising RTX and a pharmaceutically acceptable carrier has a pH in the range of 6 to 7.6. In some embodiments, the pharmaceutical formulation comprising RTX and a pharmaceutically acceptable carrier has a pH in the range of 6 to 6.4, 6.3 to 6.7, 6.4 to 6.8, 6.8 to 7.2, 7 to 7.4, or 7.2 to 7.6. In some embodiments, the pharmaceutical formulation comprising RTX and a pharmaceutically acceptable carrier has a pH of 6.5 or 7.2.

In some embodiments, the formulation comprises polysorbate 80 and dextrose. In some embodiments, the concentration of polysorbate 80 is 0.03-7% w/v. In some embodiments, the concentration of polysorbate 80 is 2-4% w/v, and/or the concentration of dextrose is 4-6% w/v. In some embodiments, the concentration of polysorbate 80 is 3% w/v, and/or the concentration of dextrose is 5% w/v. The formulation may further comprise a buffer, such as phosphate buffer (e.g., sodium phosphate buffer). In some embodiments, the concentration of phosphate buffer is 10-50 mM. In some embodiments, the concentration of phosphate buffer is 10-30 mM. In some embodiments, the concentration of phosphate buffer is 10mM. In some embodiments, the concentration of phosphate buffer is 30 mM. The formulation may have a pH in the range of 7-7.5, such as about 7.2. In some embodiments, in any of the foregoing formulations, the concentration of RTX may be 10-30 mcg/ml, such as 10 mcg/ml or 25 mcg/ml. In some embodiments, the formulation further comprises phosphate buffer, e.g., at a concentration and pH shown for phosphate buffer in Table 1. In some embodiments, the formulation further comprises NaCl, e.g., at a concentration shown for NaCl in Table 1. When both are present, the phosphate buffer and NaCl may be (but are not necessarily) present at a combination of concentrations and phosphate buffer pH shown for an individual formulation.

Exemplary formulations of RTX are shown in the following table.

**Table 1. Exemplary RTX Solution Formulations**

| **Formulation Number** | **Formulation Components** | **Component Concentration** |
|---|---|---|
| 1 | RTX | 200 mcg/mL |
| | Polysorbate 80 | 7.0% w/v |
| | Dextrose | 0.8% w/v |
| | 30 mM Phosphate Buffer w/ 0.44% NaCl | 30 mM, pH 7.2 |
| 2 | RTX | 200 mcg/mL |
| | Polyethylene Glycol 300 | 3.0% v/v |
| | Polysorbate 80 | 0.1% w/v |
| | Dextrose | 0.8% w/v |
| | 10 mM Phosphate Buffer w/ 0.73% NaCl | 10 mM, pH 6.5 |
| 3 | RTX | 200 mcg/mL |
| | Polyethylene Glycol 300 | 30.0% v/v |
| | Polysorbate 80 | 1.0% w/v |
| | 10 mM Phosphate Buffer w/ 0.86% NaCl | 10 mM, pH 6.5 |
| 4 | RTX | 200 mcg/mL |
| | Polyethylene Glycol 300 | 30.0% v/v |
| | Polysorbate 80 | 0.04% w/v |
| | 10 mM Phosphate Buffer w/ 0.88% NaCl | 10 mM, pH 6.5 |
| 5 | RTX | 200 mcg/mL |
| | Polysorbate 80 | 3.0% w/v |
| | Dextrose | 0.8% w/v |
| | 30 mM Phosphate Buffer w/ 0.54% NaCl | 30 mM, pH 7.2 |
| 6 | RTX | 200 mcg/mL |
| | Polysorbate 80 | 3.0% w/v |
| | Mannitol | 0.8% w/v |
| | 30 mM Phosphate Buffer w/ 0.54% NaCl | 30 mM, pH 7.2 |
| 7 | RTX | 200 mcg/mL |
| | Polysorbate 80 | 7.0% w/v |
| | Mannitol | 0.8% w/v |
| | 30 mM Phosphate Buffer w/ 0.45% NaCl | 30 mM, pH 7.2 |
| 8 | RTX | 200 mcg/mL |
| | Polyethylene Glycol 300 | 3.0% v/v |
| | Polysorbate 80 | 0.1% w/v |
| | Mannitol | 0.8% w/v |
| | 10 mM Phosphate Buffer w/ 0.74% NaCl | 10 mM, pH 6.5 |
| 9 | RTX | 200 mcg/mL |
| | Polyethylene Glycol 300 | 3.0% v/v |
| | Polysorbate 80 | 0.1% w/v |
| | Dextrose | 3.0% w/v |
| | 10 mM Phosphate Buffer w/ 0.34% NaCl | 10 mM, pH 6.5 |
| 10 | RTX | 200 mcg/mL |
| | Polyethylene Glycol 300 | 3.0% v/v |
| | Polysorbate 80 | 0.1% w/v |
| | Mannitol | 3.0% w/v |
| | 10 mM Phosphate Buffer w/ 0.36% NaCl | 10 mM, pH 6.5 |
| 11 | RTX | 200 mcg/mL |
| | Polysorbate 80 | 0.03% w/v |
| | Dextrose | 0.05% w/v |
| | 30 mM Phosphate Buffer w/ 0.54% NaCl | 30 mM, pH 7.2 |
| 12 | RTX | 200 mcg/mL |
| | Polysorbate 80 | 3.0% w/v |
| | Dextrose | 5.0% w/v |
| | 30 mM Phosphate Buffer w/ 0.54% NaCl | 30 mM, pH 7.2 |
| 13 | RTX | 25 mcg/mL |
| | Polysorbate 80 | 3.0% w/v |
| | Dextrose | 5.0% w/v |
| | 30 mM Phosphate Buffer w/ 0.54% NaCl | 30 mM, pH 7.2 |
| 14 | RTX | 25 mcg/mL |
| | Polysorbate 80 | 0.03% w/v |
| | Dextrose | 0.05% w/v |
| | 30 mM Phosphate Buffer w/ 0.54% NaCl | 30 mM, pH 7.2 |
| 15 | RTX | 100 mcg/mL |
| | Polysorbate 80 | 0.03% w/v |
| | Dextrose | 0.05% w/v |
| | 30 mM Phosphate Buffer w/ 0.54% NaCl | 30 mM, pH 7.2 |
| 16 | RTX | 200 mcg/mL |
| | Polysorbate 80 | 7.0% w/v |
| | Dextrose | 5.0% w/v |
| | 30 mM Phosphate Buffer w/ 0.54% NaCl | 30 mM, pH 7.2 |

In some embodiments, formulations in Table 1 include dextrose. In embodiments, the concentration of dextrose is 0.05-5% w/v. In some embodiments, the concentration of dextrose is 0.8-5% w/v. In some embodiments, the concentration of dextrose is 0.05% w/v. In some embodiments, the concentration of dextrose is 0.8% w/v. In some embodiments, the concentration of dextrose is 3.0% w/v. In some embodiments, the concentration of dextrose is 5.0% w/v.

In some embodiments, formulations in Table 1 include mannitol. In some embodiments, the concentration of mannitol is 0.8-3.0% w/v. In some embodiments, the concentration of mannitol is 0.8% w/v. In some embodiments, the concentration of mannitol is 3.0% w/v.

In some embodiments, the dextrose or mannitol is omitted from a formulation shown in Table 1.

In some embodiments, the concentration of RTX in a formulation shown in Table 1 is adjusted to any of the RTX concentrations or concentration ranges disclosed herein. For example, in some embodiments, the concentration of RTX in a formulation shown in Table 1 is adjusted to 0.3-200 mcg/ml. In some embodiments, the concentration of RTX in a formulation shown in Table 1 is 200 mcg/ml. In some embodiments, the concentration of RTX in a formulation shown in Table 1 is 0.3-100 mcg/ml. In some embodiments, the concentration of RTX in a formulation shown in Table 1 is 100 mcg/ml. In some embodiments, the concentration of RTX in a formulation shown in Table 1 is adjusted to 0.3-50 mcg/ml. In some embodiments, the concentration of RTX in a formulation shown in Table 1 is 25 mcg/ml. As another example, in some embodiments, the concentration of RTX in a formulation shown in Table 1 is adjusted to 0.3-15 mcg/ml. As another example, in some embodiments, the concentration of RTX in a formulation shown in Table 1 is adjusted to 0.5-10 mcg/ml. As another example, in some embodiments, the concentration of RTX in a formulation shown in Table 1 is adjusted to 0.6-1.5 mcg/ml. The dextrose or mannitol is omitted from any such formulation having an adjusted RTX concentration.

The formulations in Table 1 may be prepared according to the following exemplary methods, which are provided for formulations 3 and 5 but may be adapted to the other formulations by one skilled in the art. Formulation 3 may be made by adding 46 mg sodium phosphate monobasic monohydrate, 94.7 mg sodium phosphate dibasic anhydrous, and 860 mg NaCl to a 100 ml volumetric flask. 50 ml of water for injection (WFI) is added to dissolve the components in the flask, followed by addition of 1.0 g of polysorbate 80, to form the aqueous component. 20 mg of RTX is added to the aqueous component in the volumetric flask, and pH is adjusted with hydrochloric acid/sodium hydroxide to 7.2. Then 30 mL of PEG 300 is added and the solution is sonicated to dissolve the solids. It should be noted that RTX will sometimes precipitate at the interface of aqueous solution and PEG initially, but will go back into solution upon sonication. The full mixture in the flask is diluted to volume (100.00 ml) with water (WFI) and this is mixed by an inversion process. The full formulation is filtered through a 0.2 µm polytetrafluoroethylene (PTFE) filter.

Formulation 5 may be made by adding 138 mg sodium phosphate monobasic monohydrate, 284.1 mg sodium phosphate dibasic anhydrous, and 540 mg NaCl to a 100 ml volumetric flask. 50 ml of water for injection (WFI) is added to dissolve the components in the flask, followed by addition of 3.0 g of polysorbate 80, and 800 mg of dextrose to form the aqueous component. 20 mg of RTX is added the aqueous component in the volumetric flask, and pH is adjusted with hydrochloric acid/sodium hydroxide to 7.2. The solution is then sonicated to dissolve all the solids. (Alternatively, the RTX may be initially dissolved in a small volume of ethanol or DMSO, and this solution may then be added to the aqueous component.) The full mixture in the flask is diluted to volume (100.00 ml) with water (WFI) and this is mixed by an inversion process. The full formulation is filtered through a 0.2 µm PTFE filter.

A formulation according to Formulation 11 is prepared using 200 mcg RTX, 300 mcg Polysorbate 80 (using commercially-available polysorbate 80); 5.4 mg of sodium chloride, 500 mcg of dextrose, 1.38 mg sodium phosphate monobasic monohydrate, 2.84 mg sodium phosphate dibasic anhydrous, and water (WFI) to 1 mL, then pH is adjusted with hydrochloric acid/sodium hydroxide to 7.2. As noted above, the dextrose may be omitted.

A formulation according to Formulation 13 is prepared using 25 mcg RTX, 30 mg Polysorbate 80 (using commercially-available polysorbate 80); 5.4 mg of sodium chloride, 50 mg of dextrose, 1.38 mg sodium phosphate monobasic monohydrate, 2.84 mg sodium phosphate dibasic anhydrous, water (WFI) to 1 mL, then pH is adjusted with hydrochloric acid/sodium hydroxide to 7.2. As noted above, the dextrose may be omitted.

In some embodiments, the pharmaceutical formulation is in a unit dosage form. In such form, the preparation is subdivided into unit doses containing appropriate quantities of the active component. The unit dosage form can be a packaged preparation, the package containing discrete quantities of formulation, such as in vials, ampoules, or pre-loaded syringes. Also, the unit dosage form can be, e.g., a solution or a lyophilized composition for reconstitution.

Further details on techniques for formulation and administration may be found in Gennaro, A., Ed., Remington's Pharmaceutical Sciences, 18th Ed. (1990) (Mack Publishing Co., Easton, Pa.).

In some embodiments, RTX may be administered as a one-time single dose. In some embodiments, RTX is periodically administered. In some embodiments, RTX is periodically administered to a subject in need of treatment for pulmonary inflammatory disease as needed to reduce the severity of the disease.

Provided herein are composition and methods for treating pulmonary inflammatory disease, comprising administering RTX to a subject via epidural, peri-ganglionic or intra-ganglionic injection. One embodiment provides a method of treating a mammalian subject suffering from ARDS.

In exemplary embodiments, RTX can be administered to reduce the patient's symptoms, or it can be administered to counter the mechanism of the disease itself. It will be appreciated by those skilled in the art that these therapeutic objectives are often related and the treatment can be adjusted for individual patients based on various factors. These factors include the patient's age, gender, or health status, progression of pulmonary inflammatory disease, degree of dyspnea, amount of tissue damage to the patient's respiratory tract, patient smoking history, and various environmental factors (e.g., temperature, humidity and air pollution), which may contribute to the patient's condition. The patient's therapy can be adjusted depending on the dosage, timing, route of administration, and by administering other therapeutic agents simultaneously or sequentially.

### EXAMPLES

### 1. Resiniferatoxin (RTX) Ameliorates Acute Respiratory Distress Syndrome (ARDS) in Rodent Model of Lung Injury

Respiratory failure due to ARDS is one of the major causes of mortality associated with acute lung injury (ALI) including COVID-19. ALI/ARDS may be associated with acute cytokine release, pulmonary edema, and in the long term, fibrosis. The mechanisms underlying these pathological changes are not fully understood. In Example 1, a novel neural component through cardiopulmonary spinal afferents that mediates lung pathology during ALI/ARDS was studied.

Sensory neurons innervating the heart and lung enter the central nervous system by one of two routes: through the vagus nerve into the brain stem (medulla) with cell bodies residing in the nodose ganglia and directly into the spinal cord where cell bodies reside in the Dorsal Root Ganglia (DRG). Afferents are composed of elements that respond to a variety of sensory modalities such as mechanical deformation, heat, cold, pH, and inflammatory mediators. The reflex effects following stimulation of these afferents depend on the type of stimulus and the neural pathway involved. Activation of vagal afferent pathways tends to be sympatho-inhibitory and anti-inflammatory (Komeage et al. (2018) Brain, Behavior, and Immunity 73:441-449; Bonaz et al (2016) The Journal of Physiology 594:5781-5790). On the other hand, activation of spinal afferents tends to be sympathoexcitatory and pro-inflammatory (Shanks et al. (2019) Hypertension 74:910-920; Shanks et al. (2018) Physiological Reports 6:313742; Alawi et al. (2010) Pharmacol. Ther. 125:181-195; Lazar et al. (2018) Pancreas 47:110-115; Abdulla et al. (2017) Acta Physiol (Osf) 220:404-416; Wang et al. (2017) The Journal of Physiology 595:2519-2534). It was hypothesized that ablation of lung afferent innervation (thoracic spinal) by application of an ultrapotent, selective afferent neurotoxin, RTX would modify the course of the pathology including lung edema and local pulmonary inflammation associated with progressive ALI.

### Methods

*Rat Model of Lung Injury.* Rats were randomized into three groups and evaluated at 1-week post-instillation as follows: sham rats, bleomycin (Bleo)-exposed rats with saline (epidural or intra-stellate injection), and Bleo-exposed rats with RTX (epidural or intra-stellate injection). Bleo (2.5 mg/kg, ~0.15 mL) was instilled intra-tracheally to the lungs under 3% isoflurane anesthesia. Sham control rats underwent intra-tracheal instillation of saline. Animals were treated with RTX or vehicle (Veh; phosphate buffered saline) by either the epidural T1-T4 DRGs route (6 µg/ml, 10µl/per ganglia) or intra-stellate ganglia administration (50 µg/ml, 5 µl/per side) 3 days following Bleo delivery (FIG. 1A-B).

*Epidural Application of RTX.* The upper thoracic spinal afferents were ablated by epidural application of RTX. The procedure for epidural administration was essentially as described by Shanks et al (2018) Physiological Reports 6:e13742. Briefly, rats were anesthetized using 2%-3% isoflurane:oxygen mixture. Rats were placed in the prone position and a small midline incision was made in the region of the T13-L1 thoracic vertebrae. Following dissection of the superficial muscles, two small holes (approximately 2 mm x 2 mm) were made in the left and right sides of T13 vertebrae. A polyethylene catheter (PE-10) was inserted into the subarachnoid space via one hole and gently advanced about 4cm approximating the T1 level. The upper thoracic sympathetic afferent ganglia were ablated by injecting resiniferatoxin (RTX; Sigma Aldrich), an ultra-potent agonist of the TRPV1 receptor into the subarachnoid space via the catheter. RTX (1 mg; Sigma Aldrich) was dissolved in a 1:1:8 mixture of ethanol, Tween 80 (Sigma-Aldrich), and isotonic saline. The first injection of RTX (6 µg/ml, 10ul) was made at a very slow speed (~ 1 minute) to minimize the diffusion of the drug. The catheter was then pulled back to T2, T3 and T4, respectively to perform serial injections (10 µl/each) at each segment. The catheter was withdrawn and the same injections were repeated on the other side. Silicone gel was used to seal the hole in the T13 vertebra. The skin overlying the muscle were closed with a 3-0 polypropylene simple interrupted suture, and betadine was applied to the wound. For post-procedure pain management, buprenorphine (0.05 mg/kg) was subcutaneously injected immediately after surgery and twice daily for 2 days.

*Intra-stellate Injection of RTX.* Rats were anesthetized using 2%-3% isoflurane:oxygen mixture. After the trachea was cannulated mechanical ventilation was started (model 683, Harvard Apparatus, South Natick, MA). The skin from the rostral end of the sternum to the level of third rib was incised. Portions of the superficial and deep pectoral muscles and the first intercostal muscles were cut and dissected. To localize the left or right stellate ganglion, the left or right precava vein were separated with a hooked glass or steel rod laterally away from the brachiocephalic artery to expose the internal thoracic artery and the costocervical artery, which are descending branches of the right subclavian artery. Stellate ganglia and ansa subclavia are located medially to the origins of the internal thoracic and costocervical arteries. Then, RTX (5µl, 50 mg/ml) was injected into the ganglia with a 5-µl Hamilton syringe (Microliter #95, Hamilton, Reno, NV, USA.) over 30 s bilaterally. An image of this procedure is shown in **FIG. 2A-B.** Following these maneuvers, the thorax between the first and second intercostal spaces was closed with continuous 4-0 Dexon II coated braided absorbable polyglycolic acid suture and the skin was closed with 3-0 polypropylene suture and the chest evacuated. Betadine was applied to the wound and the rats were allowed to recover from the anesthesia. For post-procedure pain management, buprenorphine (0.05 mg/kg) was injected subcutaneously immediately after surgery and twice daily for 2 days.

*Blood Gas Analysis.* The artery on the ventral aspect of the rat tail was used for the collection of small amounts of blood (~0.1 mL) for analyzing arterial blood gas at day 7 post Bleo treatment. The animal was restrained with a commercial restrainer so that its tail was accessible. The tail was prepared aseptically by alternating alcohol prep pads and iodine prep pads three times and the artery was punctured using a 24 G needle. A small volume of blood (~0.1 mL) was gently aspirated into the syringe for blood gas analysis (iSTAT, Abbott, Chicago, IL, USA). After sample collection, the needle was removed, and a gauze swab was pressed firmly on the puncture site to stop bleeding.

*Cytokine Assays.* Lung and plasma cytokines were measured with R&D cytokine ELISA assays (Minneapolis, MN, USA) according to the manufacturer's instructions. Organ weights were evaluated post-mortem.

*Lung Plasma Extravasation, Tissue Extraction, and Quantification of Evans Blue.* Rats were anaesthetized with pentobarbitone (40 mg/kg). Evans Blue, 20 mg/kg (10 mg/ml, dissolved in saline + 100 IE per ml heparin) was administered intravenously. After 10 min, rats were euthanized by transcardial perfusion with PBS (0.01 M, pH 7.4). The lung was taken and first photographed. Then, lung samples were immediately weighed, placed in 2 ml of N,N'-dimethyl formamide, cut into small pieces, and heated in a 50°C water bath overnight. The lung tissues were then centrifuged (1 min, 14,000 rpm) and the Evans Blue content of the lungs in the supernatant was determined in a 96-well microplate reader (infinite M200, TECAN, Männedorf, CH, Switzerland) at 620 nm (100 µl sample per well). Extravasation of Evans Blue was expressed as mg Evans Blue per g of lung tissue, by comparing the experimental values with a known standard.

*Statistics.* Statistical evaluation was analyzed using GraphPad Prism (GraphPad Software, San Diego, CA. Version 8). Differences between treatments were determined using a Mixedeffects model for repeated-measures ANOVA. For comparison between three groups (Sham, Bleo+Veh and Bleo+RTX experiments) both Tukey and Bonferroni corrections for multiple comparisons were used.

### Results

Plasma extravasation (Evans Blue) was used to assess vascular permeability after ALI. As shown in FIG. 3A-C, Bleo-treated lungs exhibited a wide distribution of Evans Blue areas in both sides. The highest intensity of Evans Blue was shown at the medial aspect of each lung. The Evans Blue areas were reduced following epidural RTX treatment at the 7-day time point after Bleo administration.

Three pro-inflammatory tissue cytokines that were prevalent in the lung following Bleo treatment are shown in FIG. 4A-C. IL-6 (FIG. 4A), IL-1β (FIG. 4B), and IFNγ (FIG. 4C) were elevated following Bleo treatment. These cytokine levels were reduced in RTX treated rats.

Cytokine levels in response to Bleo were also reduced after epidural application of RTX (FIG. 5A-C).

Plasma extravasation in response to Bleo was reduced after stellate injection of RTX. As shown in FIG. 6A-D, there was a marked reduction in Evans Blue dye in the lung following stellate injection of RTX.

Arterial blood gas data were evaluated in rats treated with Veh vs RTX intra-stellate (FIG. 7A-H). The results show that pCO₂ was elevated (FIG. 7B) and pO₂ was reduced (FIG. 7C) as was sO₂ in Bleo and Veh treated rats (FIG. 7G). Stellate administration of RTX reversed these changes suggesting improved pulmonary function and gas exchange.

FIG. 8A-B show that IL-6 (FIG. 8A) and IL-1β (FIG. 8B) levels in lung tissue were significantly reduced after stellate RTX administration.

FIG. 9A-H show body weight (BW) and individual organ weight among groups. Compared to sham rats, wet lung weight (WLW) as well as the ratio of WLW to BW was significantly higher in the Bleo and Veh treated rats, which was significantly reduced by intra-stellate injection of RTX. The data suggests that intra-stellate injection of RTX reduces lung edema post Bleo treatment.

The data demonstrates that ablation of TRPV1 sensory afferents in the presence of ALI using RTX delivered by either of two different routes that target cardiopulmonary afferents leads to rapid reduction in lung microvascular permeability and a reduction in tissue and plasmatic inflammatory markers. While pulmonary function was not directly measured in this series of experiments, arterial blood gas data suggest an improvement in gas exchange. The improved body and reduced lung weight in rats with lung injury after receiving stellate ganglia administration of RTX suggest potential clinical benefits from reduced lung edema, and protective effects for non-pulmonary organs that would otherwise be impacted by the pulmonary triggered systemic inflammatory process.

The lung is innervated by a dual sensory system including vagal and spinal afferents. Both vagal and spinal afferent fibers are composed of group A fiber (high conduction velocity) and group C fiber (low conduction velocity) axons. These fibers and their sensory endings express a variety of membrane receptors that mediate ion channel function including traditional Na⁺, K⁺ and Ca²⁺ channels (both voltage and ligand gated). A strategy has been developed to modulate the pathological effects of TRPV1 afferent neurons. The ultrapotent neurotoxin, RTX binds avidly to the TRPV1 receptor. Upon activation, TRPV1 channels are highly permeable to calcium (Hsu et al. (1985) Journal of Applied Physiology 118:1533-1543; Brown et al. (2015) Pain 156:1018-1024). After initial stimulation, high intracellular levels of calcium mediate inhibition of neuronal function. Site-specific delivery of RTX can be used to intervene in various conditions to alleviate pain, inflammation, fibrosis and plasma extravasation. It has been shown that RTX-induced TRPV1 sensory afferent deletion can block the afferent-contained neuropeptide release and reduce inflammatory pain (Karai et al. (2004) The Jounrla of Clinical Investigation 113:1344-1352). Cardiopulmonary spinal afferents can also be targeted with RTX by either application into the epidural space at thoracic levels T1-T4 11 (with some spread to higher and lower segments) or by injection into the stellate ganglia. While DRGs are considered exclusively sensory in nature, the stellates contain soma for sympathetic efferent fibers and fibers of passage for thoracic afferents as they course through DRGs and enter the spinal cord. It should be noted that in humans the stellate ganglia can be easily identified, and that this type of transcutaneous procedure can be performed with fluoroscopic or ultrasound guidance (intra-ganglionic or nerve 'block' approach). Furthermore, intra-stellate injection requires a small volume (10 µl for bilateral injection), which reduces the risk of systemic absorption of RTX and allows a higher dose of RTX to be used for local injection.

## Claims

1. A composition comprising resiniferatoxin (RTX) for use in treating pulmonary inflammatory disease in a subject.

2. The composition for use of claim 1, comprising administering the composition to the subject epidurally, peri-ganglionically or intra-ganglionically.

3. The composition for use of claim 1 or claim 2, wherein the effective amount of RTX results in a reduction in one or more cytokines comprising IL-6, IL-1 b and/or IFNy.

4. The composition for use of any one of the preceding claims, wherein the effective amount of RTX results in improved pulmonary function.

5. The composition for use of any one of the preceding claims, wherein the effective amount of RTX results in reduced lung edema.

6. The composition for use of any one of the preceding claims, wherein the subject is an adult human.

7. The composition for use of any one of the preceding claims, wherein the RTX is administered in a dose of from about 0.1 pg to about 100 pg; suitably,
wherein the dose is from about 0.1 pg to about 1 pg, about 1 pg to about 5 pg, about 5 pg to about 10 pg, about 10 pg, to about 20 pg, about 20 pg to about 50 pg, or about 50 to about 100 pg.

8. The composition for use of any one of the preceding claims, wherein the use comprises epidural administration.

9. The composition for use of any one of claims 1 to 7, wherein the use comprises a peri-ganglionic nerve block or intra-ganglionic administration.

10. The composition for use of any one of the preceding claims, wherein the RTX is administered in a pharmaceutical formulation comprising the RTX and a pharmaceutically acceptable carrier.

11. The composition for use of claim 10, wherein the pharmaceutically acceptable carrier comprises water or saline.

12. The composition for use of claim 10 or claim 11, wherein the RTX is present in the pharmaceutical formulation at a concentration ranging from 1 pg/ml to 100 pg/ml.

13. The composition for use of claim 12, wherein the RTX is present in the pharmaceutical formulation at a concentration ranging from 1 pg/ml to 5 pg/ml, 5 pg/ml to 10 pg/ml, 10 pg/ml to 20 pg/ml, 20 pg/ml to 50 pg/ml, or 50 pg/ml to 100 pg/ml.

14. The composition for use of any one of the preceding claims, wherein the pulmonary inflammatory disease is selected from the group consisting of acute respiratory distress syndrome (ARDS), chronic obstructive pulmonary disease (COPD), pulmonary arterial hypertension (PAH), chronic inflammatory lung disease, pulmonary fibrosis, pulmonary vasculitis, pulmonary sarcoidosis, inflammation and/or infection associated with lung transplantation, acute or lung rejection and/or dysfunction, bronchitis, sinusitis, asthma, cystic fibrosis, bacterial infection, fungal infection, parasite infection, viral infection, bronchiolitis obliterans syndrome (BOS), primary ciliary dyskinesia (PCD), alveolar proteinosis, idiopathic pulmonary fibrosis (IPF), eosinophilic pneumonia, eosinophilic bronchitis, inflammation and/or infection associated with mechanical ventilation, ventilator-associated pneumonia, asbestos-related airway disorder or disease, dust-related airway disorder or disease, silicosis, and radiation or chemical agent-related airway disease or disorder, and any combination thereof.

15. The composition for use of any one of the preceding claims, wherein the pulmonary inflammatory disease is acute respiratory distress syndrome (ARDS) or chronic obstructive pulmonary disease (COPD) or pulmonary arterial hypertension (PAH) or is inflammation and/or infection associated with mechanical ventilation and/or ventilator-associated pneumonia or is associated with COVID-19.

## Patentansprüche

1. Zusammensetzung, umfassend Resiniferatoxin (RTX), zur Verwendung bei der Behandlung einer pulmonalen entzündlichen Erkrankung bei einem Patienten.

2. Zusammensetzung zur Verwendung nach Anspruch 1, umfassend die Verabreichung der Zusammensetzung an den Patienten in epiduraler, periganglionärer oder intraganglionärer Form.

3. Zusammensetzung zur Verwendung nach Anspruch 1 oder Anspruch 2, wobei die wirksame Menge an RTX zu einer Reduzierung bei einem oder mehreren Zytokinen, umfassend IL-6, IL-1 b und/oder IFNy, führt.

4. Zusammensetzung zur Verwendung nach einem der vorstehenden Ansprüche, wobei die wirksame Menge an RTX zu einer verbesserten Lungenfunktion führt.

5. Zusammensetzung zur Verwendung nach einem der vorstehenden Ansprüche, wobei die wirksame Menge an RTX zu einem reduzierten Lungenödem führt.

6. Zusammensetzung zur Verwendung nach einem der vorstehenden Ansprüche, wobei der Patient ein erwachsener Mensch ist.

7. Zusammensetzung zur Verwendung nach einem der vorstehenden Ansprüche, wobei das RTX in einer Dosis von etwa 0,1 pg bis etwa 100 pg verabreicht wird,
wobei zweckmäßigerweise die Dosis von etwa 0,1 pg bis etwa 1 pg, etwa 1 pg bis etwa 5 pg, etwa 5 pg bis etwa 10 pg, etwa 10 pg bis etwa 20 pg, etwa 20 pg bis etwa 50 pg oder etwa 50 pg bis etwa 100 pg beträgt.

8. Zusammensetzung zur Verwendung nach einem der vorstehenden Ansprüche, wobei die Verwendung eine epidurale Verabreichung umfasst.

9. Zusammensetzung zur Verwendung nach einem der Ansprüche 1 bis 7, wobei die Verwendung eine periganglionäre Nervenblockade oder eine intraganglionäre Verabreichung umfasst.

10. Zusammensetzung zur Verwendung nach einem der vorstehenden Ansprüche, wobei das RTX in einer pharmazeutischen Formulierung verabreicht wird, umfassend das RTX und einen pharmazeutisch annehmbaren Träger.

11. Zusammensetzung zur Verwendung nach Anspruch 10, wobei der pharmazeutisch annehmbare Träger Wasser oder Kochsalzlösung umfasst.

12. Zusammensetzung zur Verwendung nach Anspruch 10 oder Anspruch 11, wobei das RTX in der pharmazeutischen Formulierung in einer Konzentration im Bereich von 1 pg/ml bis 100 pg/ml vorhanden ist.

13. Zusammensetzung zur Verwendung nach Anspruch 12, wobei das RTX in der pharmazeutischen Formulierung in einer Konzentration im Bereich von 1 pg/ml bis 5 pg/ml, 5 pg/ml bis 10 pg/ml, 10 pg/ml bis 20 pg/ml, 20 pg/ml bis 50 pg/ml oder 50 pg/ml bis 100 pg/ml vorhanden ist.

14. Zusammensetzung zur Verwendung nach einem der vorstehenden Ansprüche, wobei die pulmonale entzündliche Erkrankung ausgewählt ist aus der Gruppe, bestehend aus akutem Atemnotsyndrom (ARDS), chronisch obstruktiver Lungenerkrankung (COPD), pulmonaler arterieller Hypertonie (PAH), chronischer entzündlicher Lungenerkrankung, Lungenfibrose, Lungenvaskulitis, Lungensarkoidose, einer Entzündung und/oder Infektion im Zusammenhang mit einer Lungentransplantation, akuter Lungenabstoßung und/oder Lungenfunktionsstörung, Bronchitis, Sinusitis, Asthma, Mukoviszidose, bakterieller Infektion, Pilzinfektion, parasitärer Infektion, viraler Infektion, Bronchiolitis-obliterans-Syndrom (BOS), primärer ziliärer Dyskinesie (PCD), alveolärer Proteinose, idiopathischer Lungenfibrose (IPF), eosinophiler Pneumonie, eosinophiler Bronchitis, einer Entzündung und/oder Infektion im Zusammenhang mit mechanischer Beatmung, beatmungsassoziierter Pneumonie, asbestbedingten Atemwegsstörungen oder -erkrankungen, staubbedingten Atemwegsstörungen oder - erkrankungen, Silikose sowie strahlen- oder chemikalienbedingten Atemwegserkrankungen oder -störungen und beliebigen Kombinationen davon.

15. Zusammensetzung zur Verwendung nach einem der vorstehenden Ansprüche, wobei die pulmonale entzündliche Erkrankung ein akutes Atemnotsyndrom (ARDS) oder eine chronisch obstruktive Lungenerkrankung (COPD) oder eine pulmonale arterielle Hypertonie (PAH) ist oder eine Entzündung und/oder Infektion im Zusammenhang mit einer mechanischen Beatmung und/oder einer beatmungsassoziierten Pneumonie ist oder mit COVID-19 in Zusammenhang steht.

## Revendications

1. Composition comprenant de la résinifératoxine (RTX) destinée à être utilisée dans le traitement d'une maladie inflammatoire pulmonaire chez un sujet.

2. Composition destinée à être utilisée selon la revendication 1, comprenant l'administration de la composition au sujet par voie épidurale, péri-ganglionnaire ou intra-ganglionnaire.

3. Composition destinée à être utilisée selon la revendication 1 ou la revendication 2, dans laquelle la quantité efficace de RTX conduit à une réduction d'une ou plusieurs cytokines comprenant IL-6, IL-1b et/ou IFNy.

4. Composition destinée à être utilisée selon l'une quelconque des revendications précédentes, dans laquelle la quantité efficace de RTX conduit à une fonction pulmonaire améliorée.

5. Composition destinée à être utilisée selon l'une quelconque des revendications précédentes, dans laquelle la quantité efficace de RTX conduit à une réduction de l'œdème pulmonaire.

6. Composition destinée à être utilisée selon l'une quelconque des revendications précédentes, dans laquelle le sujet est un être humain adulte.

7. Composition destinée à être utilisée selon l'une quelconque des revendications précédentes, dans laquelle la RTX est administrée en une dose d'environ 0,1 pg à environ 100 pg ; de façon appropriée,
dans laquelle la dose est comprise entre environ 0,1 pg et environ 1 pg, environ 1 pg et environ 5 pg, environ 5 pg et environ 10 pg, environ 10 pg et environ 20 pg, environ 20 pg et environ 50 pg ou environ 50 pg et environ 100 pg.

8. Composition destinée à être utilisée selon l'une quelconque des revendications précédentes, dans laquelle l'utilisation comprend l'administration épidurale.

9. Composition destinée à être utilisée selon l'une quelconque des revendications 1 à 7, dans laquelle l'utilisation comprend un bloc nerveux péri-ganglionnaire ou une administration intra-ganglionnaire.

10. Composition destinée à être utilisée selon l'une quelconque des revendications précédentes, dans laquelle la RTX est administrée dans une formulation pharmaceutique comprenant la RTX et un support pharmaceutiquement acceptable.

11. Composition destinée à être utilisée selon la revendication 10, dans laquelle le support pharmaceutiquement acceptable comprend de l'eau ou une solution saline.

12. Composition destinée à être utilisée selon la revendication 10 ou la revendication 11, dans laquelle la RTX est présente dans la formulation pharmaceutique à une concentration allant de 1 pg/ml à 100 pg/ml.

13. Composition destinée à être utilisée selon la revendication 12, dans laquelle la RTX est présente dans la formulation pharmaceutique à une concentration allant de 1 pg/ml à 5 pg/ml, de 5 pg/ml à 10 pg/ml, de 10 pg/ml à 20 pg/ml, de 20 pg/ml à 50 pg/ml ou de 50 pg/ml à 100 pg/ml.

14. Composition destinée à être utilisée selon l'une quelconque des revendications précédentes, dans laquelle la maladie inflammatoire pulmonaire est choisie dans le groupe constitué par le syndrome de détresse respiratoire aiguë (ARDS), la bronchopneumopathie chronique obstructive (COPD), l'hypertension artérielle pulmonaire (PAH), la maladie pulmonaire inflammatoire chronique, la fibrose pulmonaire, la vascularite pulmonaire, la sarcoïdose pulmonaire, l'inflammation et/ou l'infection associée à une transplantation pulmonaire, le rejet et/ou le dysfonctionnement aigu(ë) ou pulmonaire, la bronchite, la sinusite, l'asthme, la fibrose cystique, l'infection bactérienne, l'infection fongique, l'infection parasitaire, l'infection virale, la bronchiolite oblitérante (BOS), la dyskinésie ciliaire primitive (PCD), la protéinose alvéolaire, la fibrose pulmonaire idiopathique (IPF), la pneumonie éosinophile, la bronchite éosinophile, l'inflammation et/ou l'infection associée à la ventilation mécanique, la pneumonie associée à la ventilation mécanique, le trouble ou la maladie des voies respiratoires lié(e) à l'amiante, le trouble ou la maladie des voies respiratoires lié(e)s à la poussière, la silicose et la maladie ou le trouble des voies respiratoires lié(e)s aux radiations ou à un agent chimique, et une quelconque combinaison de ceux-ci.

15. Composition destinée à être utilisée selon l'une quelconque des revendications précédentes, dans laquelle la maladie inflammatoire pulmonaire est le syndrome de détresse respiratoire aiguë (ARDS), la bronchopneumopathie chronique obstructive (COPD) ou l'hypertension artérielle pulmonaire (PAH), ou est une inflammation et/ou une infection associée à la ventilation mécanique et/ou à une pneumonie associée à la ventilation assistée, ou est associée à la COVID-19.
